# EUROPEAN PATENT SPECIFICATION

(11) **EP 3 957 258 B1**
(45) Date of publication and mention of the grant of the patent: **14.08.2024**
(21) Application number: 20790687.6
(22) Date of filing: 16.04.2020
(51) Int. Cl.: A61B 17/32, A61B 17/3207, A61B 17/00, A61B 18/14, A61B 17/70, A61B 17/3205, A61B 17/16, A61B 17/22

(54) **TISSUE REMOVAL DEVICE**
GEWEBEENTFERNUNGSVORRICHTUNG
DISPOSITIF DE RETRAIT DE TISSU

(30) Priority: 16.04.2019 KR 20190044519; 18.10.2019 KR 20190130232
(43) Date of publication of application: 23.02.2022
(73) Proprietor: Labnpeople Co.,Ltd., Yangju-si, Gyeonggi-do 11477 (KR)
(72) Inventor: CHO, Sung Youn, Uijeongbu-si Gyeonggi-do 11812 (KR); CHOO, Hyun Wook, Seongnam-si Gyeonggi-do 13647 (KR); GU, Jong Su, Seoul 02813 (KR)
(74) Representative: Kador & Partner Part mbB
(86) International application number: PCT/KR2020/005055
(87) International publication number: WO 2020/213934

(56) References cited:
- WO-A1-2014/045332
- WO-A2-2012/004766
- KR-A- 20150 132 899
- KR-B1- 101 334 500
- US-A1- 2008 188 879
- US-A1- 2010 100 098
- US-A1- 2010 100 098
- US-A1- 2013 053 827
- US-A1- 2013 110 145
- US-A1- 2015 066 033
- US-A1- 2015 126 854
- US-A1- 2017 000 511
- US-A1- 2018 078 354

## Description

### Technical Field

The present disclosure relates to a tissue removal device and, more particularly, to a tissue removal device configured to be able to be inserted in a narrow space or a tubular shape inside a body such as an intervertebral foramen, a reproductive organ, and an eyeball, and to be able to easily remove a tissue that presses a nerve or causes a disease.

### Background Art

It is required to remove abnormal tissues or bones in a body in many cases of various surgical operations. For example, in surgical operations for spinal stenosis, benign prostatic hyperplasia, and adenomyosis, etc., it is required to remove not only a soft tissue, but a hard tissue in a body in some cases.

In these diseases, spinal stenosis is a degenerative disease that is caused by bond spur, buckling of a posterior longitudinal ligament, hypertrophy of a facet joint or ligament flavum, or the like. That is, spinal stenosis can be considered as a disease that is generated when a bone tissue or a soft tissue unexpectedly reproduces and presses the spinal nerves.

Various types of surgery such as pneumatic dilatation, spinal fusion, neuroplasty, and foraminotomy are used to treat this disease.

Pneumatic dilatation, which is surgery of putting a balloon in a narrowed nerve root passage using a catheter and then expanding the nerve root passage by inflating the balloon, thereby removing the adhesion state and attenuate direct pressure on a nerve, has the advantage of simplicity and quick recovery, but as limitation that it is applied to patients with slight symptoms because it is difficult to fundamentally treat an adhering portion and the possibility of return is very high.

Spinal fusion can be considered as a surgical method of preventing movement between two vertebrae to maintain a predetermined gap between the two vertebrae. This surgery is generally accompanied by wide cutting, and there is a defect that even if it is noninvasive surgery, it takes long time in comparison to other recent noninvasive surgery. Further, since implants for fixing vertebrae are inserted, the mobility of the patient is decreased by fusion of the vertebrae, so there is an effect that instability of adjacent segments increases, and high cost and long-time medical treatment are required. Accordingly, economic and physical burdens are large problems.

Neuroplasty can be considered as a surgical method of inserting a soft catheter through a vertebral canal elongated through the spine and then spraying a medicine to a diseased part that causes a pain. This surgery is noninvasive surgery and has the advantage of quick recovery, but is not a fundamental solution for the structural problem causing stricture and is difficult to be applied with physical treatment, so it has a defect that the effect of improving the conditions of illness is not large.

Foraminotomy can be considered as a surgical method of securing a nerve passage of an intervertebral foramen by scraping off a biological tissue, etc. and narrowing the intervertebral foramen using a medical instrument having a soft permeation tube. This surgery has the advantage that it is possible to perform an operation while visually checking the operation part using an endoscope, but has limitation in scraping off all of the three-dimensional tissues inside and outside an intervertebral foramen although it can remove tissues outside the intervertebral foramen, so it cannot provide a function enough for removing the tissues of all parts pressing a nerve. Further, since a medical instrument and an endoscope has to reach a diseased part, they have to be inserted in two or more directions or the diameter of the equipment is increased, which increases discomfort of a patient such as complicating the operation and increasing the recovery period.

Accordingly, the applicant(s) has proposed a tissue removal device that can minimize the defects of the types of surgery described above while increasing the advantages of existing surgery equipment, and as a relevant document, there is Korean Patent No. 10-1302453, titled 'Pecutaneous extraforaminotomy with foraminal ligament resection and instrument tools being used for the same'.

US 2010/100098 A1 discloses a tissue removal device including a plurality of bending blocks and a tissue removal unit disposed at the front end portion. It

WO 2012/004766 A2 discloses a tissue removal device comprising a tissue removal unit disposed at the front end portion.

### Disclosure

### Technical Problem

The present disclosure has been made in an effort to solve the problems described above and an objective of the present disclosure is to provide a tissue removal device of which the front end can be bent at a predetermined angle to correspond to a tissue, which presses a nerve or causes a disease in a body, when scraping the tissue.

Another objective of the present disclosure is to provide a tissue removal device of which a scraper for directly scraping a tissue, which presses a nerve, is straightly reciprocated at the end of the device by an operator.

Another objective of the present disclosure is to provide a tissue removal device that can prevent an injury of another nerve when a scraper scrapes a tissue.

Another objective of the present disclosure is to provide a tissue removal device that provides a housing type, which can be easily held by an operator, of which the front end is bent only by simple finger movement of an operator, and of which a scraper can be straightly reciprocated.

### Technical Solution

The invention is defined by claim 1. Further embodiments of the invention are defined by the dependent claims.

The present disclosure includes:
a housing; a catheter extending from the housing and configured to be inserted into the body of a patient; a bending unit including a plurality of bending blocks that is disposed at a front end portion of the catheter and at least one or more wires that control a motion direction of the bending blocks; and a tissue removal unit disposed at the front end portion of the catheter and configured to come in contact with a tissue to be removed.

Further, the bending unit may be bent with a predetermined curvature at the front end portion of the catheter.

Further, the tissue removal unit is exposed from the bending unit and brought in contact with a tissue to be removed when the bending unit is bent.

Further, the bending unit may include: a first wire configured to keeping the bending blocks aligned at the front end portion of the catheter; a second wire configured to provide tension that can bend the bending blocks; and a cap connected to a foremost bending block of the bending blocks and passing the first wire and the second wire.

Further, when the cap is pulled by the second wire and rotated on the front end portion of the catheter, the bending blocks may be bent with a predetermined curvature.

Further, the cap may be rotated at an angle of 0 - 90 degrees on the front end portion of the catheter.

Further, the tissue removal unit may include: a shaft configured to move forward or backward in the catheter and having a front end portion disposed in an internal space formed by the bending blocks; and a blade disposed at the front end of the shaft.

Further, the present disclosure may include a tension adjuster configured to provide tension to the second wire, in which the tension adjuster may include: a dial rotatably disposed in the housing and connected to the second wire; and a knob disposed on the dial and exposed downward out of the housing.

Further, the present disclosure may further include a stopper module configured to restrict rotation of the dial, in which the stopper module may include: a rotary plate disposed in the housing, having a latch, which is configured to be engaged with a gear formed at the dial, at a first longitudinal side, and having a rotary pin, which is rotatably connected to the housing, at a second longitudinal side; a pressing member integrally connected to a width-directional side of the rotary plate and exposed through a side of the housing; and an elastic member disposed in the housing and configured to elastically support a bottom of the rotary plate.

Further, the present disclosure may further include an operation unit configured to move forward or backward the shaft, in which the operation unit may include: a reciprocating block disposed to be able to reciprocate in the housing and connected to a base end of the shaft; a trigger rotatably disposed in the housing and configured to move forward or backward the reciprocating block; and a returning member configured to return the trigger into an initial state.

Further, a guide in which an assistant treatment instrument can be inserted may be disposed in the housing, and a first longitudinal side of the guide may be disposed in the housing and a second longitudinal side of the guide may be exposed rearward out of the housing.

Further, the assistant treatment instrument inserted in the guide may be inserted into a through-hole formed in the cap through the inside of the catheter.

Further, the assistant treatment instrument may include any one of a medicine transmission tube, an endoscope, and a current applier of an electrocautery.

Further, the housing may include: an accommodation part having a space therein; and a grip part extending with a predetermined curvature from a second longitudinal portion of the accommodation part and configured to be held by a palm of an operator, in which an opening that exposes the knob to the outside may be formed on a top of the second longitudinal portion of the accommodation part, and an opening that exposes the pressing member may be formed on a side of the second longitudinal portion of the accommodation part.

Further, the present disclosure may further include a connector configured to be separably coupled to the cap, in which the connector may guide a guide wire, which guides an insertion position or an insertion path of the catheter, into a through-hole of the cap which communicates with the catheter.

Further, the connector may have: a first insertion hole in which the cap is inserted; and a second insertion hole communicating with the first insertion hole and having an inner diameter that gradually decreases toward the through-hole of the cap.

Further, the present disclosure may further include an operation unit configured to move forward or backward the shaft, in which the operation unit may include: a reciprocating block disposed to be able to reciprocate in the housing and connected to a base end of the shaft; and a trigger integrally formed with the reciprocating block, exposed upward out of the housing, and configured to be straightly operated by a user.

Further, the present disclosure may include a tension adjuster configured to provide tension to the second wire, in which the tension adjuster may include: a dial rotatably disposed in the housing and connected to the second wire; and a knob disposed on the dial and exposed upward out of the housing.

The present disclosure may further include a stopper module configured to restrict rotation of the dial, in which the stopper module may include: a switch button disposed in the housing to be movable in parallel with a rotary shaft of the dial and having a longitudinal end exposed out of a side or another side of the housing to be pressed by a user; and a latch formed at the switch button, and configured to be engaged with a gear formed at the dial, depending on movement of the switch button.

### Description of Drawings

FIG. 1 is a perspective view of a tissue removal device according to an embodiment of the present disclosure;
FIG. 2 is a perspective view showing the inside of the tissue removal device shown in FIG. 1;
FIG. 3 is a side view of the tissue removal device shown in FIG. 2;
FIG. 4 is an enlarged perspective view of the part A of FIG. 1 to show the configuration of a bending unit according to an embodiment of the present disclosure;
FIG. 5 is a side view showing the state in which the bending unit of an embodiment of the present disclosure has been bent;
FIG. 6 is a perspective view showing the bending unit according to an embodiment of the present disclosure from the front;
FIG. 7 is a reference picture showing that the bending unit of the tissue removal device according to an embodiment of the present disclosure has been inserted and bent in a tubular structure;
FIG.8 is a perspective view showing that a connector according to an embodiment of the present disclosure has been coupled to a cap;
FIG. 9 is a cross-sectional view of the connector shown in FIG. 8;
FIG. 10 is a perspective view of a tissue removal device according to another embodiment of the present disclosure;
FIG. 11 is an exploded perspective view of the tissue removal device shown in FIG. 10;
FIG. 12 is a side view showing the inside of the tissue removal device shown in FIG. 10; and
FIG. 13 is an exploded perspective view showing the configuration of an operation unit and a tension adjuster of the tissue removal device shown in FIG. 10.

### Mode for Invention

The advantages and features of the present disclosure, and methods of achieving them will be clear by referring to the exemplary embodiments that will be describe hereafter in detail with reference to the accompanying drawings.

However, the present disclosure is not limited to the exemplary embodiments described hereafter and may be implemented in various ways, and the exemplary embodiments are provided to complete the description of the present disclosure and let those skilled in the art completely know the scope of the present disclosure and the present disclosure is defined by claims.

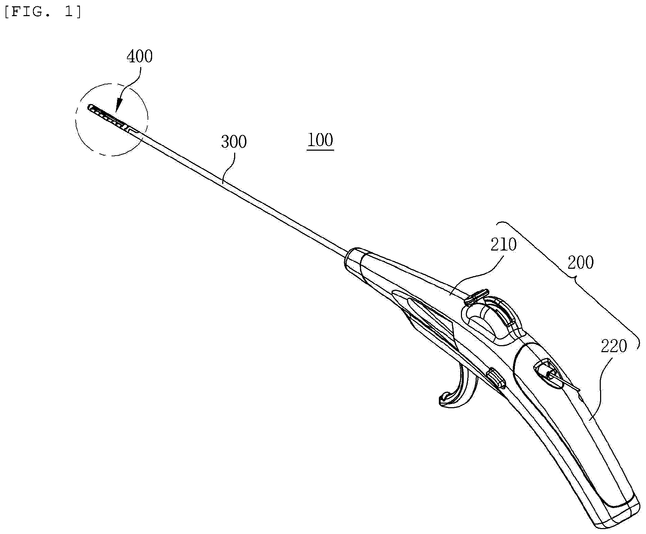

Hereafter, a tissue removal device according to an embodiment of the present disclosure will be described in detail with reference to FIGS. 1 to 7. In the description of the present disclosure, detailed description of well-known relevant functions or configurations is omitted not to make the scope of the present disclosure unclear.

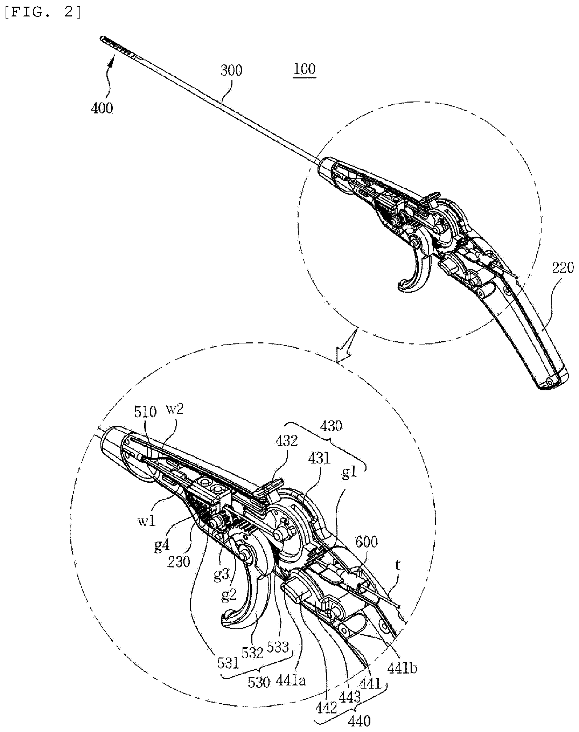

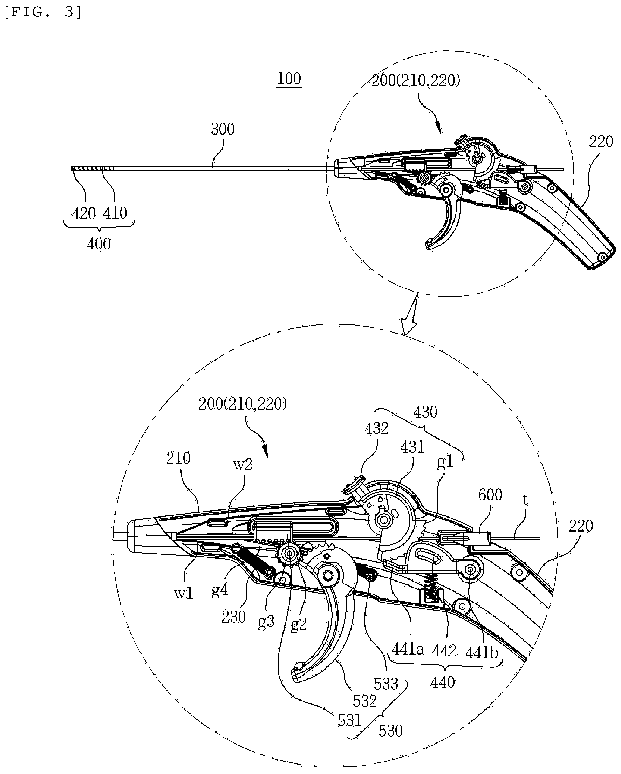

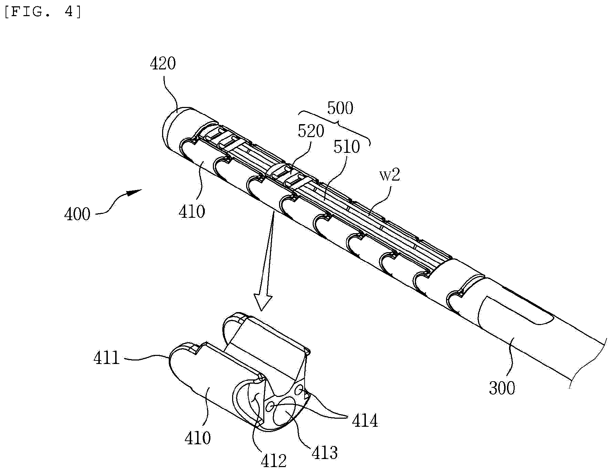

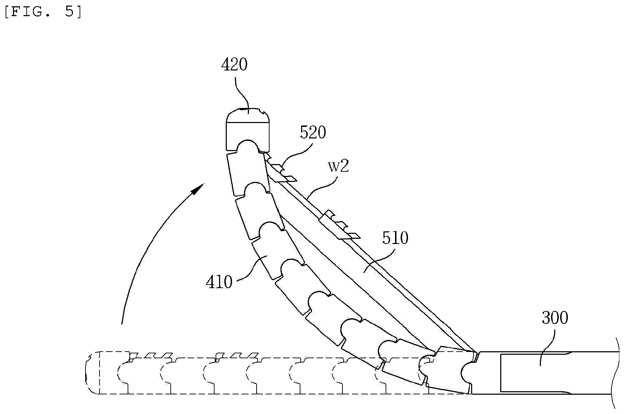

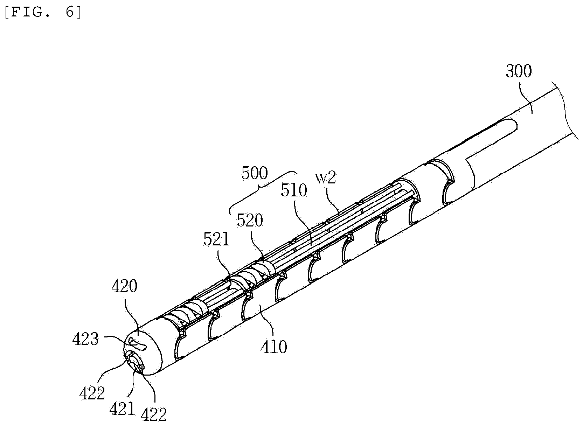

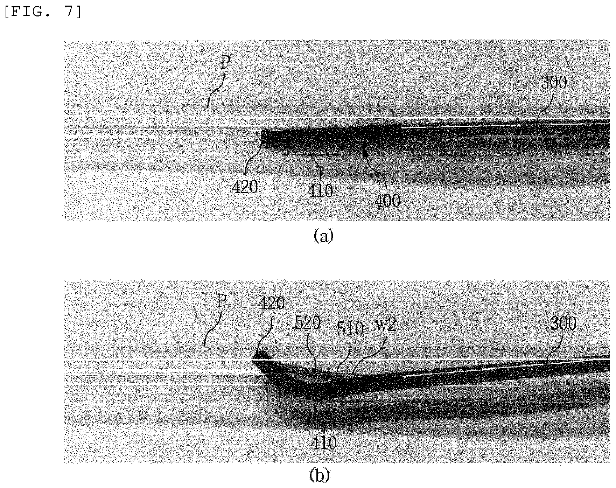

FIG. 1 is a perspective view of a tissue removal device according to an embodiment of the present disclosure, FIG. 2 a perspective view showing the inside of the tissue removal device shown in FIG. 1, FIG. 3 is a side view of the tissue removal device shown in FIG. 2, FIG. 4 is an enlarged perspective view of the part A of FIG. 1 to show the configuration of a bending unit according to an embodiment of the present disclosure, FIG. 5 is a side view showing the state in which the bending unit of an embodiment of the present disclosure has been bent, FIG. 6 is a perspective view showing the bending unit according to an embodiment of the present disclosure from the front, and FIG. 7 is a reference picture showing that the bending unit of the tissue removal device according to an embodiment of the present disclosure has been inserted and bent in a tubular structure.

As shown in FIGS. 1 to 3, a tissue removal device 100 according to an embodiment of the present disclosure may include: a housing 200; a catheter 300 extending from the housing 200 and configured to be inserted into the body of a patient; a bending unit 400 disposed at the front end portion of the catheter 300 and configured to be bent by an operator; and a tissue removal unit 500 configured to scrape a tissue while being straightly reciprocated on the bending unit 400 by an operator.

The housing 200 may have a generally gun shape to be easily held by an operator and has a space therein that can keep various components to be described below.

The catheter 300 has a hollow tube shape and has a longitudinal end disposed in the internal space of the housing and the other longitudinal portion exposed outside the housing 200.

The bending unit 400, as shown in FIGS. 2 to 4, may include: several bending blocks 410 disposed at the front end portion of the catheter 300; a first wire w1 keeping the bending blocks 410 aligned at the front end portion of the catheter 300; a second wire w2 providing tension that can bend the bending blocks 410; and a cap 420 connected to the foremost bending block 410 of the several bending blocks 410 and connected to longitudinal ends of the first wire w1 and the second wire w2.

The bending block 410, as show in FIG. 4, may be considered as blocks each having an entirely U-shaped crosssection. Accordingly, the top of the bending block 410 may be open and a space in which a shaft 510 and a blade 520 of the tissue removal unit 500 to be described below can be kept is formed in the bending block 410.

Semicircular protrusions 411 are formed at a first longitudinal end of the bending block 410 and grooves 412 having a shape corresponding to the shape of the protrusions 411 may be formed at a longitudinal second end. Accordingly, several bending blocks 410 are connected to each other by coupling the grooves 412 and the protrusions 411 of adjacent bending blocks 410, whereby the bending blocks 410 can be rotated with respect to each other.

A through-hole 413 through which an assistant treatment instrument t to be described below can pass and a through-hole 414 through which the first wire w1 passes may be formed in the bending block 410.

The first wire w1, as described above, can provide tension that can keep the bending blocks 410 aligned at the front end portion of the catheter 300.

A first longitudinal end of the first wire w1 may be connected to a tension retainer 230 disposed in the housing 200 and a second longitudinal end may be connected to the tension retainer 230 through the through-hole 414 formed in each of the bending blocks 410 and a through-hole 422 formed in the cap 420. The second longitudinal end of the first wire w1 may be considered as being connected to the bending blocks 410 and the cap 420 through the catheter 300.

The tension retainer 230 may be a coil spring, and a first longitudinal end thereof may be fixed to a fixing member formed in the housing 200 and a second longitudinal end may be connected to the first wire w1. The first wire w1 keeps tensioned by the tension retainer 230, and accordingly, the bending blocks 410 can also keep aligned and tensioned.

The second wire w2, as described above, can provide tension that can bend the bending blocks 410 at the front end portion of the catheter 200.

A first longitudinal end of the second wire w2 may be connected to a dial 431 disposed in the housing 200 and a second longitudinal end may be connected to the dial 431 through the catheter 200 and the cap 420.

The cap 420 may have a shape that can be easily inserted into the body of a patient. In an embodiment of the present disclosure, as shown in FIGS. 4 and 6, the cap 420 has a semispherical shape and is connected to the foremost bending block 510 of the several bending blocks 410.

A through-hole 421 through which the assistant treatment instrument t can pass and a through-hole 422 through which the first wire w1 can pass are also formed in the cap 420 and are respectively connected to the throughholes 413 and 414 formed in the bending blocks 410. A through-hole 423 through which the second wire w2 can pass may be further formed in the cap 420.

As shown in FIG. 5, when the front end portion of the bending unit 400 having the configuration described above is rotated toward a tissue to be scraped, the longitudinal portion of the bending unit 400 excluding the front end portion can be bent with a predetermined curvature opposite to the tissue to be scraped. That is, when the cap 420 of the bending unit 400 is pulled by the second wire w2 and rotated toward a tissue at an angle between about 0 to 90 degrees, the bending blocks 410 can be bent with a predetermined curvature opposite to the tissue.

The tissue removal unit 500 may include; a shaft 510 moving forward and backward in the catheter 200 with the front end portion disposed in the bending blocks 410; and blades 520 disposed at the front end portion of the shaft 510.

A first longitudinal end of the shaft 510 may be connected to the operation unit 530 that is disposed in the housing 200 and is described below, and a second longitudinal end may extend toward the bending unit 400 through the catheter 300. The front end portion, that is, the second longitudinal end portion of the shaft 510 may be seated in an internal space defined by the bending blocks 410, and the other longitudinal portion may be disposed in the internal space of the catheter 300 and the internal space of the housing 200.

The blades 520 may be formed on the second longitudinal portion of the shaft 510 that is exposed outside the bending unit 400. For reference, in an embodiment of the present disclosure, two blades 520 are formed with a gap therebetween on the shaft 510.

The blades 520 may be mounted on the front end portion of the shaft 510 by various existing bending methods, but may be mounted on the shaft 510 by welding not to separate from the shaft 510 when scraping a hard tissue.

A through-hole 521 through which the second wire w2 can pass when the second wire w2 bends or stretches the bending unit 400 be being pulled or returned by a tension adjuster 430 to be described below.

The through-hole 521 prevents the blades 520 from being interfered with by the second wire w2 when the blades 520 are straightly reciprocated by the shaft 510 moving forward or backward. The shaft 510 and the blades 520 are guided by the second wire w2, so they can move only straight without shaking left and right.

As shown in FIG. 5, when the bending unit 400 is bent, the tissue removal unit 500 having the configuration described above can be exposed toward a tissue to be scraped while bending at a predetermined angle from the over the bending unit 400 and can be straightly reciprocated. That is, the shaft 510 and the blades 520 are exposed toward a tissue to be scraped from the internal space of the bending blocks 410, and in this case, the second longitudinal end portion of the shaft 510 can be bent at a predetermined angle.

In this state, when the shaft 510 is moved forward or backward, the blade 520 can scrape a tissue while straightly reciprocating.

In other words, the front end portion of the shaft 510 and the blades 520 at the front end portion are not almost exposed outside the bending blocks 410 of the bending unit 400 before the bending blocks 410 are bent, but they can be completely exposed out of the internal space defined by the bending blocks 410 when the bending blocks 410 are rotated and bent in one direction.

Accordingly, the tissue removal unit 500 can easily approach a diseased part in a body and can be positioned to face a tissue to be scraped.

The bending unit 400 is not limited to the function of bending toward a tissue to be scraped such that the tissue removal unit 500 is positioned to face a tissue.

That is, the bending unit 400 can function as a protector that prevents the blades 520 from applying an injury by scraping nerves or other tissues around a tissue to be scraped when the tissue removal unit 500 scrapes the tissue to be scraped by repeatedly reciprocating.

As shown in FIG. 7(a), the bending unit 400 may be inserted in a narrow space having a tube shape and remove a tissue in a body.

For reference, FIG. 7 is a reference view for easily understanding that the catheter 300 and the bending unit 400 of the present disclosure is inserted and operated in a tube-shaped structure in a body, in which the catheter 300 and the bending unit 400 are inserted in a pipe P made of a transparent material.

The bending blocks 410, as shown in FIG. 7(b), can come in contact with the bottom inside the pipe P while bending in the narrow space of the pipe P. The cap 420 can come in contact with the top inside the pipe P.

Accordingly, the bending unit 400 is supported at two points in the tube-shaped structure, so the tissue removal unit 500 not only can be more stably moved forward and backward in a tube-shaped structure inside a body, but can more easily remove a tissue to be scraped because the contact force between the tissue removal unit 500 and the tissue is increased.

For reference, the shaft 510 may be made of a high-flexible metallic material or plastic resin material so that the front end portion can be bent with a predetermined curvature and then returned straightly. Similarly, the bending blocks 410 of the bending unit 400 may also be made of a high-flexible metallic material or plastic resin material so that they can be bent with a predetermined curvature and then returned into the initial state.

The tissue removal device 100 according to an embodiment of the present disclosure may include a tension adjuster 430 that bends the bending blocks.

The tension adjuster 430, as shown in FIGS. 2 and 3, may include: a dial 431 rotatably disposed in the housing 200 and connected to the second wire w2; and a knob 432 formed on the dial 431 and exposed upward out of the housing 200.

The dial 431 has an entirely semicircular shape and may be rotatably disposed in the housing 200. The longitudinal end of the second wire w2 can be connected and fixed to a side of the dial 431.

A first gear g1 may be circumferentially formed on the outer surface of the lower portion of the dial 431. The gear may be considered as a component engaged with a stopper module 440 to be described below.

The knob 432 may be considered as a component that is operated by an operator to rotate the dial 431, and may be formed on the outer surface of the dial 431 where the gear g1 is not formed. The knob 432, as shown in FIGS. 1 to 3, is exposed outside the housing 432 to be operated by an operator.

Accordingly, when an operator pulls the knob 432 toward himself/herself with a finger, the dial 431 is rotated in one direction and the second wire w2 connected to the side of the dial 431 can rotate upward the cap 420 of the bending unit 420 while moving. Accordingly, the bending blocks 410 connected through the first wire w2 can be bent with a predetermined curvature.

The dial 431 rotated by the operator can be stopped and maintained in the stop state by the stopper module 440.

The stopper module 440, as shown in FIGS. 2 and 3, may include: a rotary plate 441 disposed in the housing 200, having a latch 441a formed at a first longitudinal side and engaged with the gear g1 of the dial 431, and having a rotary pin 441b disposed at a second longitudinal side and rotatably connected to the housing 200; a pressing member 442 integrally connected to a width-directional side of the rotary plate and exposed through a side of the housing; and an elastic member 443 disposed in the housing 200 and elastically supporting the bottom of the rotary plate 441.

First, the rotary plate 441 may be disposed under the dial 431 and has the latch 441a, which can be engaged with the gear g1 of the dial 431, as described above, at the first longitudinal end. The rotary pin 441b rotatably disposed in the housing 200 is disposed at the longitudinal second end of the rotary plate 441.

The pressing member 442 may protrude from a plate extending perpendicularly from the width-directional side of the rotary plate 441, and as described above, may be exposed through a side of the housing 200 to be able to be operated by an operator.

The elastic member 443 may be a compression spring, and may be perpendicularly disposed in the housing 200 while elastically supporting the bottom of the rotary plate 441. That is, the lower end of the elastic member 443 is in contact with the inner side of the housing 200 and the upper end thereof is in contact with the bottom of the rotary plate 441.

Accordingly, when an operator presses the pressing member 442 exposed out of the housing 200, the first longitudinal side of the rotary plate 441 is rotated downward, and accordingly, the elastic member 443 can be compressed.

In this state, the latch 441a at the first longitudinal side of the rotary plate 441 is not in contact with the gear g1 of the dial 431, so the dial 431 can be in a rotatable state.

Then, the operator, as described above, can bend the bending blocks 410 of the bending unit 300 at a desired angle using the tension adjuster 430.

When the operator releases the pressing member 442, the compressed elastic member 443 returns into the initial state and presses the bottom of the rotary plate 441, and accordingly, the first longitudinal side of the rotary plate 441 can be rotated upward. Accordingly, the latch 441a of the rotary plate 441 is engaged with the gear g1 of the dial 431, so the dial 431 can be maintained in a stopped state.

Meanwhile, the tissue removal device 100 according to an embodiment of the present disclosure may include an operation unit 530 configured to move forward and backward the shaft 510.

The operation unit 530, as shown in FIGS. 2 and 3, may include: a reciprocating block 531 disposed to be able to straightly reciprocate in the housing 200 and connected to a base end of the shaft 510; a trigger 532 rotatably disposed in the housing 200 and configured to move forward or backward the reciprocating block 531; and a returning member 533 configured to return the trigger 531 into the initial state.

The reciprocating block 531 may be seated in a guide groove formed on the inner side of the housing 200 and may be connected and fixed to the second longitudinal end of the shaft 510.

The trigger 532 may be considered as a component that is operated by an operator. The upper portion of the trigger 532 may be disposed in the housing and the other longitudinal portion may be exposed downward out of the housing 200. A gear g2 is formed at the upper portion of the trigger 532 that is disposed in the housing 200. The gear g2 can be engaged with a pinion gear g3 rotatably disposed in the housing 200. The pinion gear g3 can be engaged with a rack gear g4 formed on the bottom of the reciprocating block 531.

The returning member 533 may be a coil spring. A first longitudinal end of the returning member 533 may be connected to the upper portion of the trigger 532 that is disposed in the housing 200 and a second longitudinal end may be connected to the inner side of the housing 200.

Accordingly, when an operator pulls the trigger 532 exposed downward out of the housing 200 toward himself/herself, the gear g2 of the trigger 532 rotates the pinion gear g3 in one direction, and accordingly, the reciprocating block 531 can be moved backward. Accordingly, the shaft 510 connected to the reciprocating block 531 is moved backward by the movement distance of the reciprocating block 531. In this case, the coil spring that is the returning member 533 is stretched.

On the other hand, when the operator releases the trigger 532, the retuning member 533 pulls the upper portion of the trigger 532 while contracting. Accordingly, the longitudinal portion of the trigger 532 that is exposed downward out of the housing 200 can rotate away from the operator and return into the initial state.

Further, the gear g2 of the trigger 532 rotates the pinion gear g3 in another direction, and accordingly, the reciprocating block 531 and the shaft 510 can be moved forward.

When the operator repeatedly holds and pulls, and releases the trigger 532, it is possible to remove a tissue to be scraped while the front end portion of the shaft 510 on which the blades 520 are mounted repeatedly move forward and backward on the bending unit 400.

Meanwhile, the tissue removal device 100 according to an embodiment of the present disclosure may further include a guide 600 that may be inserted in the assistant treatment instrument t to remove a tissue causing a pain at a diseased part in a body and apply an assistant treatment to the diseased part.

The assistant treatment instrument t may be a tube-shaped medicine transmission tube that transmits (injects) a medicine to a diseased part in a body or may be a tube-shaped endoscope that enables an operator to visually check a diseased part in a body. Alternatively, the assistant treatment instrument t may be a current applier of an electric stimulator or an electrocautery. That is, the assistant treatment instrument t may be considered as a tube-shaped member made of a flexible material of well-known surgery devices that are used to additionally treat a diseased part.

The guide 600 has a through-hole through which the assistant treatment instrument t can pass. A first longitudinal side of the guide 600 may be disposed in the housing 200 and a second longitudinal side may be exposed rearward from the housing 200 to face an operator.

The assistant treatment instrument t inserted in the through-hole of the guide 600 can be inserted into the through-hole 421 formed in the cap 420 through the inside of the catheter 300.

For reference, it is important that the assistant treatment instrument t is not interfered with by various components in the housing 200 when the assistant treatment instrument t inserted in the guide 600 is inserted into the through-hole 421 of the cap 420 through the catheter 300. Accordingly, a through-hole through which the assistant treatment instrument t inserted in the guide 600 can pass may be formed in each of the dial 431 of the tension adjuster 430 and the reciprocating block 531 of the operation unit 530.

According to this configuration, since the front end of the assistant treatment instrument t can be positioned at the front end of the bending unit 400, accurately, in the through-hole 421 formed in the cap 420, a process of removing a tissue and a process of performing an additional treatment are simultaneously performed, so a patient can be effectively treated.

Meanwhile, the housing 200 according to an embodiment of the present disclosure may have a structural shape that enables an operator to simply operate the tension adjuster 430, the operation unit 530, and the stopper module 440.

The housing 200, as shown in FIGS. 1 to 3, may include an accommodation part 210 having a space therein, and a grip part 220 extending from the accommodation part 210 with a predetermined curvature and configured to be held by a palm of an operator.

The accommodation part 210 has an internal space in which the various components described above can be disposed.

An opening that exposes the knob 432 of the tension adjuster 430 may be formed on the top of a second longitudinal portion of the accommodation part 210. The second longitudinal portion of the accommodation part 210 may be a longitudinal portion of the accommodation part 210 which is close to the grip part 220 that is held by a palm of an operator.

Similarly, an opening that exposes the pressing member 442 of the stopper module 440 may be formed on a side of the second longitudinal portion of the accommodation part 210.

The reason of forming the openings, which can expose the knob 432 of the tension adjuster 430 and the pressing member 442 of the stopper module 440, respectively, at the second longitudinal portion of the accommodation part 210 which is close to the grip part 220 is for enabling an operator to easily operate the tension adjuster 430 and the stopper module 440 through simple finger movement with the grip part 200 held by hand. In other words, the reason is for enabling a user to easily operate the tension adjuster 430 or the stopper module 440 with a thumb or an index finger with the grip part 220 surrounded in a palm.

An opening that exposes the trigger 532 to the outside may be formed on the bottom of the accommodation part, and accordingly, an operator holding the grip part 220 can easily operate the trigger 532 with his/her index finger or middle finger.

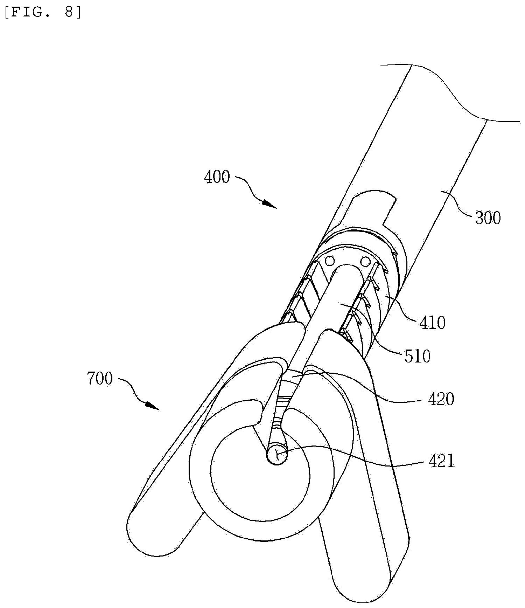

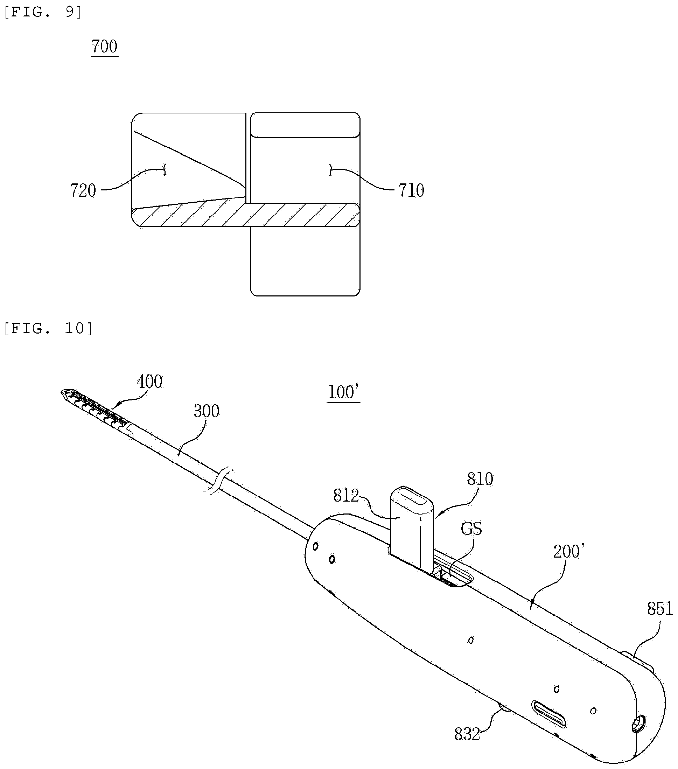

Meanwhile, the tissue removal device 100 according to an embodiment of the present disclosure, as shown in FIGS. 8 and 9, may further include a connector 700.

The connector 700 is separably coupled to the cap 420 and functions as a guide so that an operator can easily insert a guide wire into the through-hole 421 formed in the cap 420.

The guide wire, unlike the assistant treatment instrument t described above, can be primarily inserted into the cap 410 and then inserted into the catheter 300 through the shaft 510 before the catheter 300 is inserted into a body.

The guide wire guides the insertion position or the insertion path of the catheter, so it may be inserted into the catheter 300 by an operator before the catheter 300 is inserted into a body. However, since the diameter of the through-hole 421 formed in the cap 410 is 1cm or less, it is difficult for an operator to insert the guide wire into the through-hole 421. Accordingly, the connector 700 can be coupled to the cap 420 and can enable an operator to easily insert the guide wire.

The connector 700, as shown in FIG. 9, may have a first insertion hole 710 in which the cap 410 is inserted and a second insertion hole 720 communicating with the first insertion hole 710 and having an inner diameter gradually decreasing toward the through-hole 421 of the cap 420.

Accordingly, when an operator inserts the guide wire into the second insertion hole 720 with the cap 420 inserted in the first insertion hole 710 of the connector 700, the end of the guide wire can be easily inserted into the through-hole 420 of the cap 420 while being guided by the inner side of the second insertion hole 720. When the guide wire is fully inserted, the connector 700 can be separated from the cap 420.

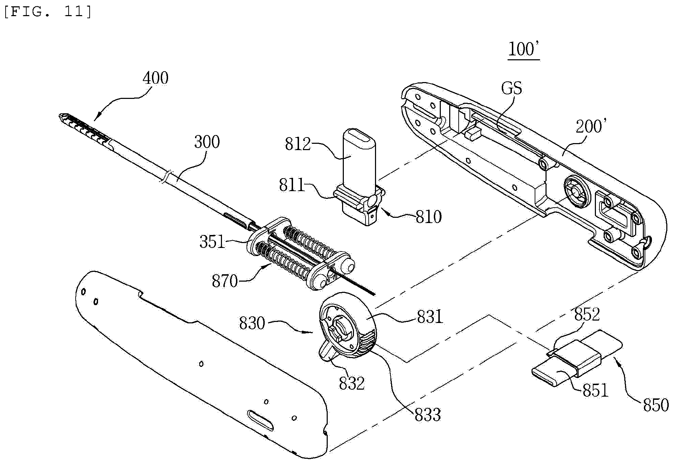

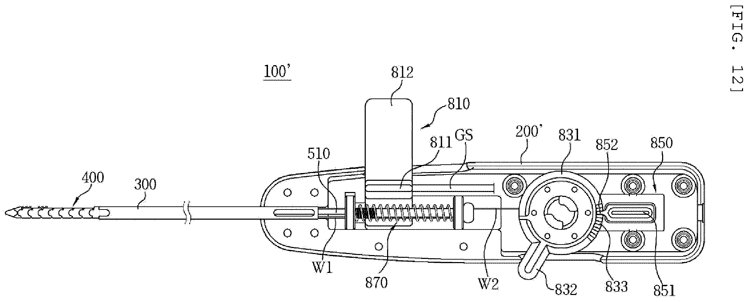

Hereafter, a tissue removal device 100' according to another embodiment of the present disclosure will be described in detail with reference to FIGS. 10 to 13.

The tissue removal device 100' according to another embodiment of the present disclosure, as compared with the tissue removal device 100 according to an embodiment of the present disclosure, is different only in the configuration of a housing 200', an operation unit 810, a tension adjuster 830, and a stopper module 850, but is the same in the other configuration.

Accordingly, only the operation unit 810, the tension adjuster 830, and the stopper module 850 of the tissue removal device 100' according to another embodiment of the present disclosure are described hereafter.

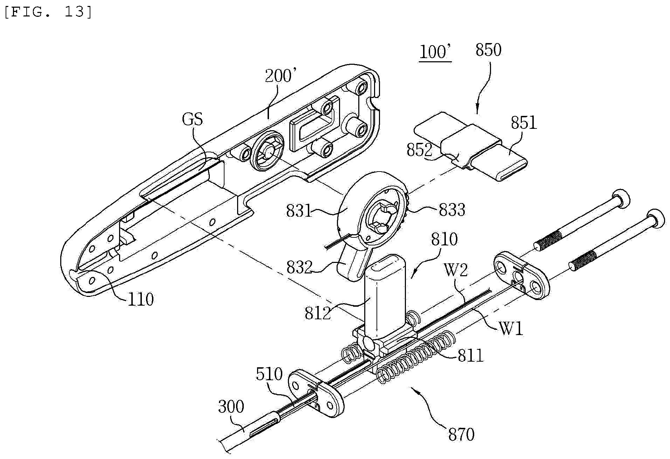

The operation unit 810 according to another embodiment of the present disclosure, as shown in FIGS. 10 and 13, may include: a reciprocating block 811 disposed to be able to straightly reciprocate in the housing 200' and connected to the base end of the shaft 300; and a trigger 812 integrally formed with the reciprocating block 811, exposed upward out of the housing 200', and configured to the straightly operated by an operator.

First, the housing 200' is disposed in parallel with the longitudinal direction of the catheter 300 and may have a rod shape so that an operator can easily surround and hold it with a palm.

The reciprocating block 811 of the operation unit 810 can be straightly moved by an operator in the state in which it is seated a guide slot GS formed on the inner side of the housing 200'.

The trigger 812, as described above, may be integrally formed with the reciprocating block 811 and may be exposed to the outside through an opening formed on the top of the housing 200'. That is, the lower end of the trigger 812 may be connected to the reciprocating block 811 and the upper end may be exposed upward out of the housing 200' .

Accordingly, an operator can bend or stretch the front end portion of the catheter by straightly reciprocating the trigger 812 exposed upward out of the housing 200'.

The tension adjuster 830, as shown in FIGS. 10 to 13, may include: a dial 831 rotatably disposed in the housing 200' and connected to the second wire w2; and a knob 831 disposed on the dial 832 and exposed downward out of the housing 200'.

The dial 831 of the tension adjuster 830 may have an entirely disc shape and may be rotatably mounted on a rotary shaft disposed in the housing 200'.

The knob 832 may be integrally connected to the dial 831 and may be exposed downward out of the housing 200' in this state. Accordingly, an operator can adjust the tension of the second wire w2 using the knob 832 exposed downward out of the housing 200'. For example, when an operator pulls the knob 832 toward himself/herself, the dial 831 pulls the second wire w2 toward the operator while rotating in one direction. On the contrary, when the knob 832 is returned into the initial state, the dial 832 can return the second wire w2 into the initial state while rotating in another direction.

The stopper module 850, as shown in FIGS. 10 to 13, may include: a switch button 851 disposed in the housing to be movable in parallel with the rotary shaft of the dial 831 and having a longitudinal end exposed out of a side or another side of the housing 200' to be pressed by a user; and a latch 852 integrally formed with the switch button 851, and configured to be engaged with a gear 833 formed at the dial 831, depending on movement of the switch button 851.

The stopper module 850 having this configuration serves to maintain the degree of bending of the bending unit 400 by preventing rotation of the dial 831 after an operator adjusts the degree of bending of the bending unit 400 by rotating the dial 831. That is, when a user presses the switch button 851 such that the latch 852 and the teeth 832 formed at the dial 831 are engaged with each other, rotation of the dial 831 can be restricted.

Meanwhile, the reference number '870' shown in FIGS. 10 to 13 may be a tension retainer that retains tension of the first wire w1.

Although detailed embodiments according to the present disclosure were described above, various modifications are possible without departing from the scope of the present disclosure.

Accordingly, the scope of the present disclosure should not be limited to the embodiments described above, and should be defined by the claims.

### Industrial Applicability

The present disclosure can be applied and sold in various medical industries for treating, preventing, and attenuating a disease of a patient and rehabilitating a patient.

## Claims

1. A tissue removal device (100) comprising:
a housing (200) ;
a catheter (300) extending from the housing (200) and configured to be inserted into the body of a patient;
a bending unit (400) including a plurality of bending blocks (410) that is disposed at a front end portion of the catheter (300) and at least one or more wires (w1, w2) that control a motion direction of the bending blocks; and
a tissue removal unit (500) disposed at the front end portion of the catheter (300) and configured to come in contact with a tissue to be removed,
wherein the bending unit (400) can be bent with a predetermined curvature at the front end portion of the catheter (300) and
**characterised in that**
the tissue removal unit (500) is exposed from the bending unit (400) and can be brought in contact with a tissue to be removed when the bending unit (500 is bent.

2. The tissue removal device (100) of claim 1, wherein the bending unit (400) includes:
a first wire (w1) configured to keeping the bending blocks (410) aligned at the front end portion of the catheter;
a second wire (w2) configured to provide tension that can bend the bending blocks (410); and
a cap (420) connected to a foremost bending block (410) of the bending blocks (410) and passing the first wire (w1) and the second wire (w2).

3. The tissue removal device (100) of claim 2, wherein when the cap (420) is pulled by the second wire (w2) and rotated on the front end portion of the catheter (300) , the bending blocks (410) are bent with a predetermined curvature.

4. The tissue removal device (100) of claim 3, wherein the cap (420) is rotated at an angle of 0 ~ 90 degrees on the front end portion of the catheter (300) .

5. The tissue removal device (100) of claim 1, wherein the tissue removal unit (500) includes:
a shaft (510)configured to move forward or backward in the catheter (300) and having a front end portion disposed in an internal space formed by the bending blocks; and
a blade (520) disposed at the front end of the shaft (510) .

6. The tissue removal device (100) of claim 2, further comprising a tension adjuster (430) configured to provide tension to the second wire (w2),
wherein the tension adjuster (430) includes:
a dial (431) rotatably disposed in the housing (200) and connected to the second wire (w2); and
a knob (432) disposed on the dial (431) and exposed upward out of the housing.

7. The tissue removal device (100) of claim 6, further comprising a stopper module (440) configured to restrict rotation of the dial (431),
wherein the stopper module (440)includes:
a rotary plate (441) disposed in the housing (200), having a latch (441a), which is configured to be engaged with a gear (g1) formed at the dial (431), at a first longitudinal side, and having a rotary pin (441b), which is rotatably connected to the housing (200), at a second longitudinal side;
a pressing member (442) connected to a width-directional side of the rotary plate (441) and exposed through a side of the housing (200); and
an elastic member (443) disposed in the housing (200) and configured to elastically support a bottom of the rotary plate (441) .

8. The tissue removal device (100) of claim 5, further comprising an operation unit (530) configured to move forward or backward the shaft (510),
wherein the operation unit (530) includes:
a reciprocating block (531) disposed to be able to reciprocate in the housing (200) and connected to a base end of the shaft (510);
a trigger (532) rotatably disposed in the housing (200) and configured to move forward or backward the reciprocating block (531); and
a returning member (533) configured to return the trigger (532)into an initial state.

9. The tissue removal device (100) of claim 2, wherein a guide (600) in which an assistant treatment instrument (t) can be inserted is disposed in the housing (200), and
a first longitudinal side of the guide (600) is disposed in the housing 8200) and a second longitudinal side of the guide is exposed rearward out of the housing.

10. The tissue removal device (100) of claim 9, wherein the assistant treatment instrument (t) inserted in the guide (600) is inserted into a through-hole (421) formed in the cap (420)through the inside of the catheter (300).

11. The tissue removal device (100) of claim 9, wherein the assistant treatment instrument (t) includes any one of a medicine transmission tube, an endoscope, and a current applier of an electrocautery.

12. The tissue removal device (100) of claim 6, wherein the housing (200)comprises:
an accommodation part (210) having a space therein; and
a grip part (220) extending with a predetermined curvature from a second longitudinal portion of the accommodation part (210) and configured to be held by a palm of an operator,
wherein an opening that exposes the knob (432) to the outside is formed on a top of the second longitudinal portion of the accommodation part (210), and an opening that exposes the pressing member (442) is formed on a side of the second longitudinal portion of the accommodation part (210).

13. The tissue removal device (100) of claim 2, further comprising a connector (700) configured to be separably coupled to the cap (420),
wherein the connector (700) guides a guide wire, which guides an insertion position or an insertion path of the catheter (300), into a through-hole (421) of the cap which communicates with the catheter (300).

## Patentansprüche

1. Gewebeentnahmevorrichtung (100), umfassend:
ein Gehäuse (200),
einen Katheter (300), der sich von dem Gehäuse (200) erstreckt und konfiguriert ist, um in den Körper eines Patienten eingeführt zu werden.
eine Biegeeinheit (400), die eine Vielzahl von Biegeblöcken (410), die an einem vorderen Endteil des Katheters (300) angeordnet sind, und wenigstens einen oder mehrere Drähte (w1, w2), die eine Bewegungsrichtung der Biegeblöcke steuern, umfasst, und
eine Gewebeentnahmeeinheit (500), die an dem vorderen Endteil des Katheters (300) angeordnet ist und konfiguriert ist, um in einen Kontakt mit einem zu entnehmenden Gewebe zu kommen,
wobei die Biegeeinheit (400) mit einer vorbestimmten Krümmung an dem vorderen Endteil des Katheters (300) gebogen werden kann,
**dadurch gekennzeichnet, dass** die Gewebeentnahmeeinheit (500) von der Biegeeinheit (400) freiliegt und in einen Kontakt mit einem zu entnehmenden Gewebe gebracht werden kann, wenn die Biegeeinheit (500) gebogen wird.

2. Gewebeentnahmevorrichtung (100) nach Anspruch 1, wobei die Biegeeinheit (400) umfasst:
einen ersten Draht (w1), der konfiguriert ist, um die Biegeblöcke (410) an dem vorderen Ende des Katheters ausgerichtet zu halten,
einen zweiten Draht (w2), der konfiguriert ist, um eine Spannung vorzusehen, die die Biegeblöcke (410) biegen kann, und
eine Kappe (420), die mit einem vordersten Biegeblock (410) der Biegeblöcke (410) verbunden ist und zu der sich der erste Draht (w1) und der zweite Draht (w2) erstrecken.

3. Gewebeentnahmevorrichtung (100) nach Anspruch 2, wobei, wenn die Kappe (420) durch den zweiten Draht (w2) gezogen wird und an dem vorderen Endteil des Katheters (300) gedreht wird, die Biegeblöcke (410) mit einer vorbestimmten Krümmung gebogen werden.

4. Gewebeentnahmevorrichtung (100) nach Anspruch 3, wobei die Kappe (420) mit einem Winkel von 0 ~ 90 Grad an dem vorderen Endteil des Katheters (300) gedreht wird.

5. Gewebeentnahmevorrichtung (100) nach Anspruch 1, wobei die Gewebeentnahmeeinheit (500) umfasst:
eine Welle (510), die konfiguriert ist, um sich nach vorne oder nach hinten in dem Katheter (300) zu bewegen, und einen vorderen Endteil aufweist, der in einem durch die Biegeblöcke gebildeten Innenraum angeordnet ist, und
eine Klinge (520), die an dem vorderen Ende der Welle (510) angeordnet ist.

6. Gewebeentnahmevorrichtung (100) nach Anspruch 2, die weiterhin einen Spannungseinsteller (430) umfasst, der konfiguriert ist, um eine Spannung für den zweiten Draht (w2) vorzusehen,
wobei der Spannungseinsteller (430) umfasst:
eine Drehscheibe (431), die drehbar in dem Gehäuse (200) angeordnet und mit dem zweiten Draht (w2) verbunden ist, und
einen Knopf (432), der an der Drehscheibe (431) angeordnet und nach oben aus dem Gehäuse heraus freiliegt.

7. Gewebeentnahmevorrichtung (100) nach Anspruch 6, die weiterhin ein Stoppermodul (440) umfasst, das konfiguriert ist, um eine Drehung der Drehscheibe (431) zu beschränken,
wobei das Stoppermodul (440) umfasst:
eine Drehplatte (441), die in dem Gehäuse (200) angeordnet ist und an einer ersten Längsseite eine Klinke (441a) aufweist, die konfiguriert ist, um mit einem an der Drehscheibe (431) ausgebildeten Zahnrad (g1) einzugreifen, und an einer zweiten Längsseite einen Drehstift (441b) aufweist, der drehbar mit dem Gehäuse (200) verbunden ist,
ein Drückglied (442), das mit einer Breitenrichtungsseite der Drehplatte (441) verbunden ist und durch eine Seite des Gehäuses (200) freiliegt, und
ein elastisches Glied (443), das in dem Gehäuse (200) angeordnet ist und konfiguriert ist, um einen Boden der Drehplatte (441) elastisch zu halten.

8. Gewebeentnahmevorrichtung (100) nach Anspruch 5, die weiterhin eine Betätigungseinheit (530) umfasst, die konfiguriert ist, um die Welle (510) nach vorne oder nach hinten zu bewegen,
wobei die Betätigungseinheit (530) umfasst:
einen sich hin und her bewegenden Block (531), der derart angeordnet ist, dass er sich in dem Gehäuse (200) hin und her bewegen kann, und mit einer Basis der Welle (510) verbunden ist,
einen Auslöser (532), der drehbar in dem Gehäuse (200) angeordnet ist und konfiguriert ist, um den sich hin und her bewegenden Block (531) nach vorne und nach hinten zu bewegen, und
ein Rückstellglied (533), das konfiguriert ist, um den Auslöser (532) zu einem Ausgangszustand zurückzuversetzen.

9. Gewebeentnahmevorrichtung (100) nach Anspruch 2, wobei eine Führung (600), in die ein Hilfsbehandlungsinstrument (t) eingesteckt werden kann, in dem Gehäuse (200) angeordnet ist, und
eine erste Längsseite der Führung (600) in dem Gehäuse (200) angeordnet ist und eine zweite Längsseite der Führung nach hinten aus dem Gehäuse freiliegt.

10. Gewebeentnahmevorrichtung (100) nach Anspruch 9, wobei das in die Führung (600) eingesteckte Hilfsbehandlungsinstrument (5) in ein Durchgangsloch (421) in der Kappe (420) durch das Innere des Katheters (300) eingesteckt wird.

11. Gewebeentnahmevorrichtung (100) nach Anspruch 9, wobei das Hilfsbehandlungsinstrument (t) einen Medizinverabreichungsschlauch, ein Endoskop oder einen Stromanleger für eine Elektrokauterisation umfasst.

12. Gewebeentnahmevorrichtung (100) nach Anspruch 6, wobei das Gehäuse (200) umfasst:
einen Aufnahmeteil (210) mit darin einem Raum, und
einen Greifteil (220), der sich mit einer vorbestimmten Krümmung von einem zweiten Längsteil des Aufnahmeteils (210) erstreckt und konfiguriert ist, um durch eine Handfläche eines Bedieners gehalten zu werden,
wobei eine Öffnung, die den Knopf (432) nach außen freilegt, an einer oberen Seite des zweiten Längsteils des Aufnahmeteils (210) ausgebildet ist, und eine Öffnung, die das Drückglied (442) freilegt, an einer Seite des zweiten Längsteils des Aufnahmeteils (210) ausgebildet ist.

13. Gewebeentnahmevorrichtung (100) nach Anspruch 2, die weiterhin einen Verbindungsteil (700) umfasst, der konfiguriert ist, um trennbar mit der Kappe (420) gekoppelt zu werden,
wobei der Verbindungsteil (700) einen Führungsdraht, der eine Einführposition oder einen Einführpfad des Katheters (300) führt, in ein mit dem Katheter (300) verbundenes Durchgangsloch (421) der Kappe führt.

## Revendications

1. Dispositif d'ablation (100) de tissu, comprenant :
un boîtier (200) ;
un cathéter (300) s'étendant à partir du boîtier (200) et configuré pour être inséré dans le corps d'un patient ;
une unité de pliage (400) comprenant une pluralité de blocs de pliage (410), qui est disposée à une portion d'extrémité avant du cathéter (300) et au moins un ou plusieurs fils (w1, w2) qui commandent une direction de mouvement des blocs de pliage ; et
une unité d'ablation de tissu (500) disposée à la portion d'extrémité avant du cathéter (300) et configurée pour entrer en contact avec un tissu à ablater,
l'unité de pliage (400) pouvant être pliée avec une courbure prédéterminée à la portion d'extrémité avant du cathéter (300) et
**caractérisé en ce que** l'unité d'ablation de tissu (500) est exposée par rapport à l'unité de pliage (400) et peut être mise en contact avec un tissu à ablater lorsque l'unité de pliage (500) est pliée.

2. Dispositif d'ablation (100) de tissu selon la revendication 1 dans lequel l'unité de pliage (400) comprend :
un premier fil (w1) configuré pour maintenir les blocs de pliage (410) alignés à la portion d'extrémité avant du cathéter ;
un deuxième fil (w2) configuré pour fournir une tension qui peut plier les blocs de pliage (410) ; et
un capuchon (420) connecté à un bloc de pliage (410) le plus en avant parmi les blocs de pliage (410) et passant le premier fil (w1) et le deuxième fil (w2).

3. Dispositif d'ablation (100) de tissu selon la revendication 2, dans lequel, lorsque le capuchon (420) est tiré par le deuxième fil (w2) et tourné sur la portion d'extrémité avant du cathéter (300), les blocs de pliage (410) sont pliés avec une courbure prédéterminée.

4. Dispositif d'ablation (100) de tissu selon la revendication 3, dans lequel le capuchon (420) est tourné à un angle de 0 - 90 degrés sur la portion d'extrémité avant du cathéter (300).

5. Dispositif d'ablation (100) de tissu selon la revendication 1, dans lequel l'unité d'ablation de tissu (500) comprend :
une tige (510) configurée pour se déplacer vers l'avant ou vers l'arrière dans le cathéter (300) et ayant une portion d'extrémité avant disposée dans un espace intérieur formé par les blocs de pliage ; et
une lame (520) disposée au niveau de l'extrémité avant de la tige (510).

6. Dispositif d'ablation (100) de tissu selon la revendication 2, comprenant en outre un dispositif de réglage de tension (430) configuré pour fournir une tension au deuxième fil (w2),
le dispositif de réglage de tension (430) comprenant :
un cadran (431) disposé de manière rotative dans le boîtier (200) et connecté au deuxième fil (w2) ; et
un bouton (432) disposé sur le cadran (431) et exposé vers le haut hors du boîtier.

7. Dispositif d'ablation (100) de tissu selon la revendication 6, comprenant en outre un module d'arrêt (440) configuré pour limiter la rotation du cadran (431),
le module d'arrêt (440) comprenant :
une plaque rotative (441) disposée dans le boîtier (200), comportant un verrou (441a) qui est configuré pour être engagé avec un pignon (g1) formé sur le cadran (431), sur un premier côté longitudinal, et ayant une broche rotative (441b) qui est reliée de manière rotative au boîtier (200), sur un deuxième côté longitudinal ;
un élément de pression (442) relié à un côté orienté en largeur de la plaque rotative (441) et exposé à travers un côté du boîtier (200) ; et
un élément élastique (443) disposé dans le boîtier (200) et configuré pour soutenir élastiquement un fond de la plaque rotative (441).

8. Dispositif d'ablation (100) de tissu selon la revendication 5, comprenant en outre une unité opérationnelle (530) configurée pour déplacer la tige (510) vers l'avant ou vers l'arrière,
l'unité opérationnelle (530) comprenant :
un bloc à mouvement alternatif (531) disposé de manière à pouvoir effectuer des mouvements alternatifs dans le boîtier (200) et relié à une extrémité de base de la tige (510) ;
une gâchette (532) disposée de manière rotative dans le boîtier (200) et configurée pour déplacer vers l'avant ou vers l'arrière le bloc à mouvement alternatif (531) ; et
un élément de rappel (533) configuré pour ramener la gâchette (532) dans un état initial.

9. Dispositif d'ablation (100) de tissu selon la revendication 2, un guide (600) dans lequel peut être inséré un instrument de traitement auxiliaire (t) étant disposé dans le boîtier (200), et
un premier côté longitudinal du guide (600) étant disposé dans le boîtier (200) et un deuxième côté longitudinal du guide étant exposé vers l'arrière hors du boîtier.

10. Dispositif d'ablation (100) de tissu selon la revendication 9, l'instrument de traitement auxiliaire (t) inséré dans le guide (600) étant inséré dans un trou traversant (421) formé dans le capuchon (420) à travers l'intérieur du cathéter (300).

11. Dispositif d'ablation (100) de tissu selon la revendication 9, l'instrument de traitement auxiliaire (t) comprenant un élément quelconque parmi un tube de transmission de médicaments, un endoscope et un applicateur de courant d'un électrocautère.

12. Dispositif d'ablation (100) de tissu selon la revendication 6, le boîtier (200) comprenant :
une partie de logement (210) comportant en elle un espace ; et
une partie de préhension (220) s'étendant avec une courbure prédéterminée à partir d'une deuxième portion longitudinale de la partie de logement (210) et configurée pour être tenue par une paume de main d'un opérateur,
une ouverture exposant le bouton (432) vers l'extérieur étant formée sur un haut de la deuxième portion longitudinale de la partie de logement (210), et une ouverture exposant l'élément de pression (442) étant formée sur un côté de la deuxième portion longitudinale de la partie de logement (210).

13. Dispositif d'ablation (100) de tissu selon la revendication 2, comprenant en outre un connecteur (700) configuré pour être couplé de manière dissociable au capuchon (420),
le connecteur (700) guidant un fil de guidage, qui guide une position d'insertion ou un chemin d'insertion du cathéter (300), dans un trou traversant (421) du capuchon qui communique avec le cathéter (300).
